# EUROPEAN PATENT APPLICATION

(11) **EP 4 537 876 A1**
(43) Date of publication of application: **16.04.2025**
(21) Application number: 24824381.8
(22) Date of filing: 24.07.2024
(51) Int. Cl.: A61M 5/32, A61M 5/50, A61M 5/178

(54) **SAFE SELF-DESTRUCTION SYRINGE**

(30) Priority: 01.09.2023 CN 202311129385
(71) Applicant: Luo, Shichun, Luzhou, Sichuan 646100 (CN)
(72) Inventor: Luo, Shichun, Luzhou, Sichuan 646100 (CN)
(74) Representative: Grünecker Patent- und Rechtsanwälte PartG mbB
(86) International application number: PCT/CN2024/107168
(87) International publication number: WO 2025/044609

(57) **Abstract**

The present disclosure discloses a safety self-destruction syringe. An upper end of a needle structure of the safety self-destruction syringe is provided with a first hook buckle and a second hook buckle, and during an injection process, a first lug boss of the needle structure limits the retraction of the needle structure into a needle cylinder to achieve safe injection. After the injection is completed, a first inner hole and a second inner hole of a sealing plug are respectively fitted with the first hook buckle and the second hook buckle, an inclined surface on the second hook buckle abuts against a second convex ring of the sealing plug, while there is an avoidance space for the abutment of the second convex ring below the first hook buckle, so that a central axis of the needle structure is deflected from a central axis of the needle cylinder and can be pulled into the needle cylinder with a push rod to achieve self-destruction. In addition, various operations may be smoothly completed by various parts of the safety self-destruction syringe under the condition of completely getting rid of a lubricating effect of lubricating oil or silicone oil, and the removal of the silicone oil for the syringe reduces the risk of biological reactions which may be caused by these substances, thereby significantly improving the biocompatibility of the product.

## Description

### Technical Field

The present disclosure relates to the technical field of syringes, and more particularly relates to a safety self-destruction syringe.

### Background Art

After injection is completed by a syringe, a needle tip may be stained with body fluids or tissues of a patient, and the needle tip is also relatively sharp, thereby being relatively inconvenient to discard and treat, and increasing the risk of infection or puncture injuries to medical personnel and cleaning personnel.

A self-destruction syringe is generally in the form of retracting a needle into a needle cylinder, and a push rod in the needle cylinder pushes a sealing plug to enable the sealing plug to extrude a liquid medicine to the needle, so as to achieve injection. Since a front end of the sealing plug is provided with a buckling structure capable of being fitted with a tail end of the needle, the sealing plug is tightly buckled with the needle after the injection is completed, and when the push rod is pulled back, the sealing plug drags the needle to achieve the retraction of the needle into the needle cylinder. With this design, there is an unavoidable contradiction in use, that is, during the injection process of the needle, the needle is subjected to resistance from skin tissues, so that the needle has the tendency to move into the needle cylinder, if the needle retracts at this time, liquid leakage of the needle cylinder may be caused, thereby resulting in medical malpractice; and on the other hand, when the injection is completed, the sealing plug is tightly buckled with a tail portion of the needle, and then the push rod needs to be pulled to drag the needle to a retracted state at this time, but a force for pulling back the push rod cannot be excessive. Firstly, the sealing plug is generally made of silica gel, and after being buckled with the tail portion of the needle, a structure of the sealing plug cannot bear such a large tensile force. Secondly, the force for pulling back the push rod should not be excessive, so as to facilitate the operation of the medical personnel. In order to achieve the effect of being convenient for the medical personnel to use, a static frictional force required for preventing the retraction of the needle during the injection process of the needle is generally greater than the tensile force required for pulling back the push rod for the retraction of the needle.

In order to balance the above contradictions, the existing retractable self-destruction syringes have relatively complicated structures, product quality which is difficult to ensure, and not good actual using effects, so that the following problems often occur:
1. the sealing effect between the needle and the front end of the needle cylinder is not good, so that the front end of the needle cylinder is likely to leak liquid during the injection process;
2. after the injection is completed by the needle, during the process of pulling back the push rod, the fitting between the sealing plug and the tail end of the needle is not firm, so that the needle cannot be pulled back to retract into the needle cylinder; and
3. when the needle is pulled back to retract into the needle cylinder, the needle is likely to be pushed forwards with the push rod again and then pushed out of the needle cylinder again, thereby not achieving complete self-destruction.

### Summary of the Invention

In view of this, it is an object of the present disclosure to provide a safety self-destruction syringe which has the features of being safe in injection, and relatively easy to pull back a needle into a needle cylinder for self-destruction.

The technical solution adopted by the present disclosure to solve the technical problem thereof is as follows:
a safety self-destruction syringe includes a needle cylinder, a push rod disposed in the needle cylinder, a sealing plug disposed at a lower end of the push rod and having elasticity, and a needle structure mounted at a lower end of the needle cylinder, wherein the needle structure includes a first hook buckle disposed at an upper end and a second hook buckle disposed below the first hook buckle and located on an opposite side, an inclined surface is formed between the second hook buckle and an upper end surface of the needle, a first lug boss is disposed directly below the same side of the second hook buckle, an inner wall of the needle cylinder is provided with a first convex ring capable of being clamped on the lug boss to limit the movement of the needle structure towards a direction of the needle cylinder, a lower end of the sealing plug is provided with a first inner hole capable of being fitted with the first hook buckle and provided with a second inner hole capable of being fitted with the second hook buckle below the first inner hole, a second convex ring is disposed between the first inner hole and the second inner hole, an avoidance space capable of allowing the second convex ring to abut below the first hook buckle is formed below the first hook buckle, and the second convex ring can abut against the inclined surface, so that a central axis of the needle structure is deflected from a central axis of the needle cylinder.

The needle structure below the lug boss is a conical body with a diameter gradually decreasing from top to bottom, sealing rings are disposed outside the conical body, and the number of the sealing rings is at least two.

A liquid medicine chamber is formed between the sealing plug and the needle structure, an upper end of the needle structure is provided with an injection chamber communicated with the liquid medicine chamber, and a chamber cross section of the injection chamber gradually increases from top to bottom.

A plurality of sealing convex rings in interference fit with an inner side wall of the needle cylinder are disposed outside the sealing plug.

A guide conical surface is formed between the first hook buckle and an end surface of the upper end of the needle structure.

The lower end of the push rod is provided with a third hook buckle, an annular barb side edge is formed on an outer side of the third hook buckle, and an upper end of the sealing plug is provided with a third inner hole fitted with the third hook buckle.

The needle cylinder includes a needle cylinder chamber in which the sealing plug is located and a needle chamber for mounting the needle structure, wherein an inner diameter of the needle cylinder chamber is greater than an inner diameter of the needle chamber, and a stepped surface is formed between the needle cylinder chamber and the needle chamber.

The needle structure includes a needle, and when the needle structure is located in the needle cylinder chamber, the stepped surface can abut against the needle to limit downward movement of the needle.

The present disclosure has at least the following beneficial effects.
1. The liquid suction and injection processes of the syringe are the same as those of an ordinary syringe. After the injection is completed, firstly, the first hook buckle is embedded into the first inner hole of the sealing plug, the second convex ring abuts against the inclined surface located on the opposite side of the first hook buckle, where the inclined surface is subjected to a force, while there is the avoidance space below the first hook buckle on the opposite side, and the second convex ring abuts below the first hook buckle, so that the first hook buckle is firmly embedded into the first inner hole. At this time, the needle structure has the tendency to be inclined towards the direction of the first hook buckle, and since the sealing plug has the elasticity, as the sealing plug continues to move downwards, the second hook buckle is embedded into the second inner hole of the sealing plug, the buckling and fitting between the sealing plug and the needle structure are completed, then the push rod is pulled upwards, one edge of the sealing plug drives the needle structure to gradually move upwards, and one edge of the inclined surface at the upper end (right side) of the needle structure is subjected to the elastic force of the abutment of the second convex ring, so that the needle structure is inclined around an intersection point of the central axis and the upper end surface of the needle structure, thereby resulting in that the lug boss of the needle structure is disengaged from the first convex ring of the needle chamber, that is, the first convex ring can no longer clamp the lug boss to limit the upward movement of the needle structure, and finally, the needle structure smoothly retracts into the needle cylinder chamber as the push rod is pulled upwards.
   This design has the following benefits: firstly, the design of the lug boss and the first convex ring effectively prevents the needle from retracting due to the resistance of skin and tissues during the injection process, so as to achieve safe injection; secondly, the first hook buckle and the second hook buckle ensure tight fitting between the sealing plug and the needle structure during the process of pulling back the push rod; and thirdly, the contradictions during the using process of the conventional retractable self-destruction syringe are solved, and during the injection process, the needle can bear greater resistance from the skin and tissues without retraction. After the injection is completed, only a relatively small tensile force is required for pulling back the needle to complete the retraction and self-destruction of the needle.
2. During the retraction process of the needle structure, the optimized design of the needle structure and the sealing plug makes it easier for the needle structure to form a relatively stable fitting relationship with the sealing plug, and this process can be easily achieved without the need for lubricating oil or silicone oil; and then for the needle structure, during the process of pulling back the push rod, when the lug boss of the needle structure is disengaged from the first convex ring of the needle chamber, that is, the first convex ring can no longer clamp the lug boss to limit the upward movement of the needle structure, the needle structure can be lightly pulled away from the needle chamber, and this structural optimization enables this process to also be lightly achieved without the need for lubricating oil or silicone oil; and
   various operations may be smoothly completed by various parts of the safety self-destruction syringe under the condition of completely getting rid of a lubricating effect of lubricating oil or silicone oil, and the removal of the silicone oil for the syringe not only reduces the potential residue of chemical substances, but also reduces the risk of biological reactions which may be caused by these substances, thereby significantly improving the biocompatibility of the product.
3. A first barb enables the first hook buckle to be tightly buckled with the first inner hole, a second barb enables the second hook buckle to be tightly buckled with the second inner hole, and the guide conical surface formed between the first hook buckle and the end surface of the upper end of the needle structure makes it easier for the first hook buckle to be embedded into the first inner hole.
4. A second lug boss is used for abutting against an upper surface of the first convex ring to limit downward movement of the needle structure, so as to prevent the needle structure from falling off the needle chamber.
5. With the design of a plurality of sealing rings, on the one hand, the liquid medicine is further prevented from flowing out of the needle cylinder, and on the other hand, the static frictional force between the needle structure and the needle cylinder is increased. The sealing rings are designed to be outside the needle structure which is a conical body, when the needle structure is subjected to downward pressure, the larger the static frictional force is, the less likely the needle structure is to retract due to resistance, and when the needle structure is subjected to an upward tensile force, the smaller the static frictional force is, the more likely the needle structure is to be pulled back.
6. A chamber volume of the injection chamber gradually increases from top and bottom, so that when the injection is started, greater liquid pressure extruded by the sealing plug to flow into the injection chamber from the liquid medicine chamber is slowed down, and the pressure of the fluid at the needle on an internal environment of a human body during the injection is buffered.
7. A plurality of sealing convex rings can improve the sealing performance of the sealing plug, and on the other hand, decrease the frictional resistance from the inner wall of the needle cylinder when the push rod is pushed and pulled, so that the pushing and pulling of the push rod can be lightly achieved without the need for the effect of the silicone oil, and the removal of the silicone oil for the syringe is further achieved.
8. The barb side edge of the third hook buckle of the push rod enhances the tightness during fitting between the push rod and the sealing plug.
9. When the needle structure is pulled into the needle cylinder chamber, the needle structure is in a deflected state, and a tail end of the needle abuts against the stepped surfaces of the needle cylinder chamber and the needle chamber, so that the needle structure can no longer protrude, so as to complete the self-destruction of the needle.

### Brief Description of the Drawings

FIG. 1 is a cross-sectional view of a safety self-destruction syringe;
FIG. 2 is a partial cross-sectional view of the safety self-destruction syringe;
FIG. 3 is a schematic structural view of a needle structure;
FIG. 4 is a partial cross-sectional view of a needle cylinder;
FIG. 5 is a partial cross-sectional view of a sealing plug fitted with the needle structure; and

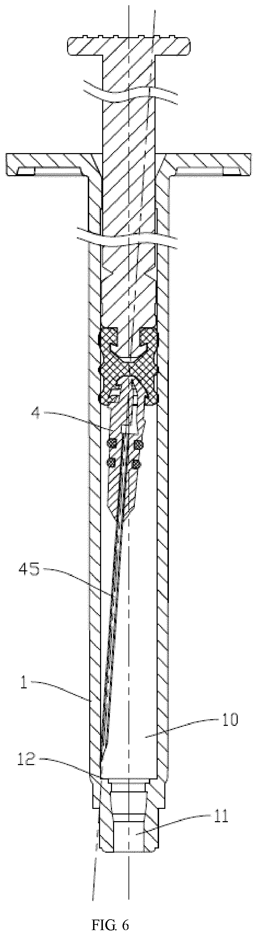

FIG. 6 is a cross-sectional view of the needle structure pulled back into the needle cylinder chamber with the sealing plug.

### Detailed Description of the Invention

In order that the technical problems to be solved, the technical solutions to be adopted and the technical effects to be achieved by the present disclosure can be more clear, the technical solutions of the present disclosure will be further described by way of specific embodiments with reference to the accompanying drawings. It should be understood that the specific examples described herein are illustrative of the present disclosure only and are not restrictive of the present disclosure. In addition, it should also be noted that for ease of description, only some, but not all, of the relevant aspects of the present disclosure are shown in the accompanying drawings.

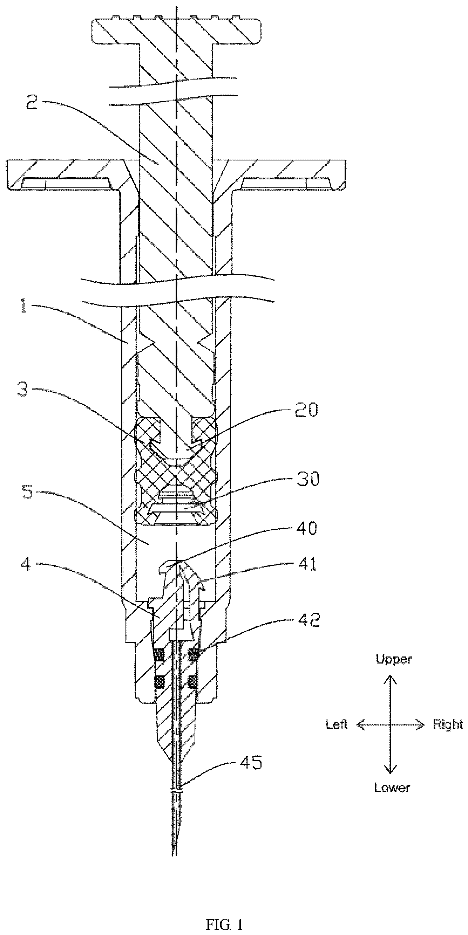

With reference to FIG. 1, FIG. 2 and FIG. 4, the present disclosure provides a safety self-destruction syringe, including a needle cylinder 1, a push rod 2 disposed in the needle cylinder 1, a sealing plug 3 disposed at a lower end of the push rod 2 and connected to the push rod 2 and having elasticity, and a needle structure 4 mounted at a lower end of the needle cylinder 1, wherein the needle structure 4 includes a first hook buckle 40 disposed at an upper end and a second hook buckle 41 disposed below the first hook buckle 40 and located on an opposite side, an inclined surface 410 is formed between the second hook buckle 41 and an upper end surface of the needle structure 4, a first lug boss 43 is disposed directly below the same side of the second hook buckle 41, an inner wall of the needle cylinder 1 is provided with a first convex ring 110 capable of being clamped on the lug boss 43 to limit the movement of the needle structure 4 towards a direction of the needle cylinder 1, a lower end of the sealing plug 3 is provided with a first inner hole 301 capable of being fitted with the first hook buckle 40 and provided with a second inner hole 302 capable of being fitted with the second hook buckle 41 below the first inner hole 301, a second convex ring 300 is disposed between the first inner hole 301 and the second inner hole 302, an avoidance space 47 capable of allowing the second convex ring 300 to abut below the first hook buckle 40 is formed below the first hook buckle 40, and the second convex ring 300 can abut against the inclined surface 410, so that a central axis of the needle structure 4 is deflected from a central axis of the needle cylinder 1.

Further, the design of the lug boss 43 and the first convex ring 110 effectively prevents the needle from retracting due to the resistance of skin and tissues during the injection process, so as to achieve safe injection; the first hook buckle 40 and the second hook buckle 41 ensure tight fitting between the sealing plug 3 and the needle structure 4 during the process of pulling back the push rod 2; and the contradictions during the using process of the conventional retractable self-destruction syringe are solved by the safety self-destruction syringe, and during the injection process, the needle can bear greater resistance from the skin and tissues without retraction. After the injection is completed, only a relatively small tensile force is required for pulling back the needle to complete the retraction and self-destruction of the needle.

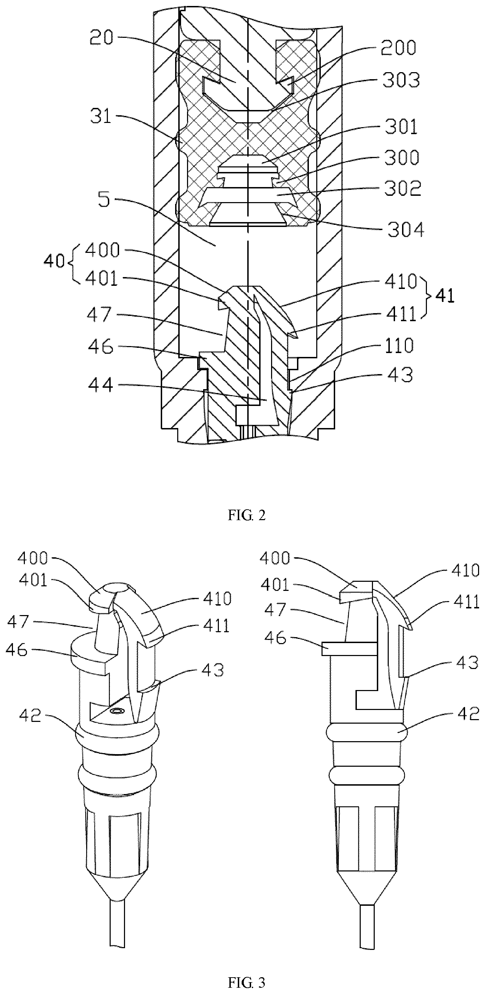

With reference to FIG. 2 or FIG. 3, the first hook buckle 40 includes a first barb 401 and a guide conical surface 400 disposed on the first barb 401, and the second hook buckle 41 includes a second barb 411 disposed at a lower end of the inclined surface 410.

With reference to FIG. 2, a second lug boss 46 capable of abutting against the upper surface of the first convex ring 110 is disposed directly below the same side of the avoidance space 47, and the second lug boss 46 can limit the downward movement of the needle structure 4 and prevent the needle structure 4 from falling off the needle chamber 11.

With reference to FIG. 3, the needle structure 4 below the lug boss 43 is a conical body with a diameter gradually decreasing from top to bottom, sealing rings 42 are disposed outside the conical body, and the number of the sealing rings 42 is at least two. Preferably, two sealing rings 42 are disposed at different upper and lower positions outside the needle structure 4, respectively, and the diameter of the sealing ring 42 disposed below is less than that of the sealing ring 42 disposed above.

Further, with the design of the sealing rings 42, on the one hand, the liquid medicine is further prevented from flowing out of the needle cylinder 1, and on the other hand, the static frictional force between the needle structure 4 and the needle cylinder 1 is increased. The sealing rings 42 are designed to be outside the needle structure 4 which is a conical body, when the needle structure 4 is subjected to downward pressure, the larger the static frictional force is, the less likely the needle structure 4 is to retract due to resistance, and when the needle structure 4 is subjected to an upward tensile force, the smaller the static frictional force is, the more likely the needle structure 4 is to be pulled back.

With reference to FIG. 2, a liquid medicine chamber 5 is formed between the sealing plug 3 and the needle structure 4, an upper end of the needle structure 4 is provided with an injection chamber 44 communicated with the liquid medicine chamber 5, and a chamber cross section of the injection chamber 44 gradually increases from top to bottom, so that when the injection is started, greater liquid pressure extruded by the sealing plug 4 to flow into the injection chamber 44 from the liquid medicine chamber 5 is slowed down, and the pressure of the fluid at the needle 45 on an internal environment of a human body during the injection is buffered.

With reference to FIG. 2, a plurality of sealing convex rings 31 in interference fit with an inner side wall of the needle cylinder 1 are disposed outside the sealing plug 3. The plurality of sealing convex rings 31 can improve the sealing performance of the sealing plug 3, and on the other hand, decrease the frictional resistance from the inner wall of the needle cylinder 1 when the push rod 2 is pushed and pulled.

Preferably, with reference to FIG. 2, a guide conical surface 400 is formed between the first hook buckle 40 and an end surface of the upper end of the needle structure 4, and the guide conical surface 400 makes it easier for the first hook buckle 40 to be embedded into the first inner hole 301.

With reference to FIG. 2, the lower end of the push rod 2 is provided with a third hook buckle 20, an annular barb side edge 200 is formed on an outer side of the third hook buckle 20, an upper end of the sealing plug 3 is provided with a third inner hole 303 fitted with the third hook buckle 20, and the barb side edge 200 of the third hook buckle 20 of the push rod 2 enhances the tightness during fitting between the push rod 2 and the sealing plug 3.

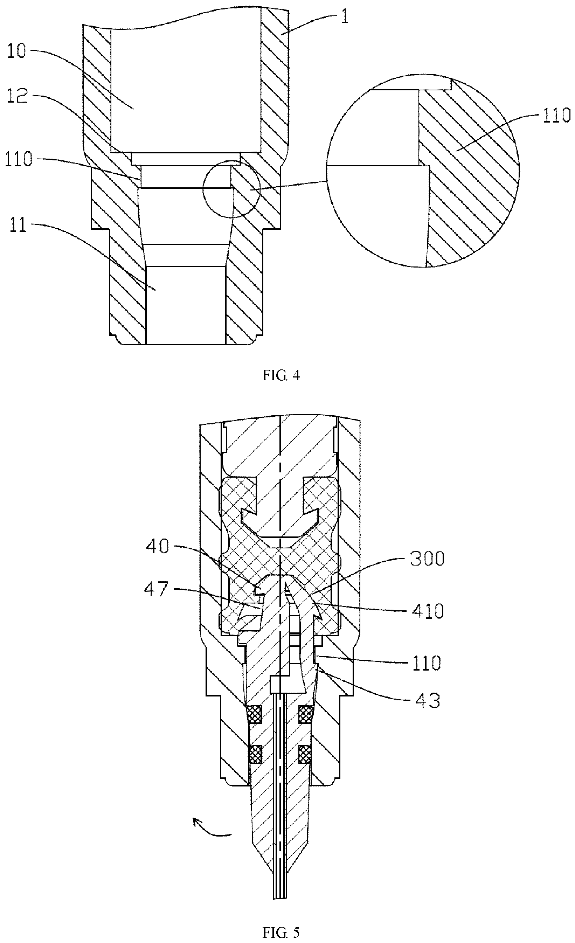

With reference to FIG. 4, the needle cylinder 1 includes a needle cylinder chamber 10 in which the sealing plug 3 is located and a needle chamber 11 for mounting the needle structure 4, wherein an inner diameter of the needle cylinder chamber 10 is greater than that of the needle chamber 11, and a stepped surface 12 is formed between the needle cylinder chamber 10 and the needle chamber 11. The needle structure 4 includes a needle 45, and when the needle structure 4 is pulled into the needle cylinder chamber 10, the needle structure 4 is in a deflected state, and a tail end of the needle 45 abuts against the stepped surfaces 12 of the needle cylinder chamber 10 and the needle chamber 11, so that the needle structure 4 can no longer protrude, so as to complete the self-destruction of the needle.

The using process and the principle of the safety self-destruction syringe are as follows.

The liquid suction and injection processes of the syringe are the same as those of an ordinary syringe. After the injection is completed, firstly, the first hook buckle 40 is embedded into the first inner hole 301 of the sealing plug 3, the second convex ring 300 abuts against the inclined surface 410 located on the opposite side of the first hook buckle 40, with reference to FIG. 5, the inclined surface is subjected to a force, while there is the avoidance space 47 below the first hook buckle 40 on the opposite side, and the second convex ring 300 abuts below the first hook buckle 40, so that the first hook buckle 40 is firmly embedded into the first inner hole 301. At this time, the needle structure 4 has the tendency to be inclined towards the direction (clockwise direction in FIG. 5) of the first hook buckle 40, and since the sealing plug 3 has the elasticity, as the sealing plug 3 continues to move downwards, the second hook buckle 41 is embedded into the second inner hole 302 of the sealing plug 3, the buckling and fitting between the sealing plug 3 and the needle structure 4 are completed, then the push rod 2 is pulled upwards, one edge of the sealing plug 3 drives the needle structure 4 to gradually move upwards, and one edge of the inclined surface 410 at the upper end (right side) of the needle structure 4 is subjected to the elastic force of the abutment of the second convex ring 300, so that the needle structure 4 is inclined around an intersection point of the central axis and the upper end surface of the needle structure 4, thereby resulting in that the lug boss 43 of the needle structure 4 is disengaged from the first convex ring 110 of the needle chamber 11, that is, the first convex ring 110 can no longer clamp the lug boss 43, so that the needle structure 4 can move upwards, and finally, the needle structure 4 smoothly retracts into the needle cylinder chamber 10 as the push rod 2 is pulled upwards.

## Claims

1. A safety self-destruction syringe includes a needle cylinder (1), a push rod (2) disposed in the needle cylinder (1), a sealing plug (3) disposed at a lower end of the push rod (2) and having elasticity, and a needle structure (4) mounted at a lower end of the needle cylinder (1), wherein the needle structure (4) includes a first hook buckle (40) disposed at an upper end and a second hook buckle (41) disposed below the first hook buckle (40) and located on an opposite side, an inclined surface (410) is formed between the second hook buckle (41) and an upper end surface of the needle (4), a first lug boss (43) is disposed directly below the same side of the second hook buckle (41), an inner wall of the needle cylinder (1) is provided with a first convex ring (110) capable of being clamped on the lug boss (43) to limit the movement of the needle structure (4) towards a direction of the needle cylinder (1), a lower end of the sealing plug (3) is provided with a first inner hole capable (301) of being fitted with the first hook buckle (40) and provided with a second inner hole (302) capable of being fitted with the second hook buckle (41) below the first inner hole (301), a guide conical hole (304) is disposed below the second inner hole (302), a second convex ring (300) is disposed between the first inner hole (301) and the second inner hole (302), an avoidance space (47) capable of allowing the second convex ring (300) to abut below the first hook buckle (40) is formed below the first hook buckle (40), and the second convex ring (300) can abut against the inclined surface (410), so that a central axis of the needle structure (4) is deflected from a central axis of the needle cylinder.

2. A safety self-destruction syringe according to claim 1,the first hook buckle (40) includes a first barb (401) and a guide conical surface (400) disposed on the first barb (401), and the second hook buckle (41) includes a second barb (411) disposed at a lower end of the inclined surface (410).

3. A safety self-destruction syringe according to claim 1, the second lug boss (46) capable of abutting against the upper surface of the first convex ring (110) is disposed directly below the same side of the avoidance space (47), and the second lug boss (46) can limit the downward movement of the needle structure (4).

4. A safety self-destruction syringe according to claim 1, the needle structure (4) below the lug boss (43) is a conical body with a diameter gradually decreasing from top to bottom, sealing rings (42) are disposed outside the conical body, and the number of the sealing rings (42) is at least two.

5. A safety self-destruction syringe according to claim 1, a liquid medicine chamber (5) is formed between the sealing plug (3) and the needle structure (4), an upper end of the needle structure (4) is provided with an injection chamber (44) communicated with the liquid medicine chamber (5), and a chamber cross section of the injection chamber (44) gradually increases from top to bottom.

6. A safety self-destruction syringe according to claim 1, a plurality of sealing convex rings (31) in interference fit with an inner side wall of the needle cylinder (1) are disposed outside the sealing plug (3).

7. A safety self-destruction syringe according to claim 1, the lower end of the push rod (2) is provided with a third hook buckle (20), an annular barb side edge (200) is formed on an outer side of the third hook buckle (20), and an upper end of the sealing plug (3) is provided with a third inner hole fitted with the third hook buckle (20).

8. A safety self-destruction syringe according to claim 1, the needle cylinder (1) includes a needle cylinder chamber (10) in which the sealing plug (3) is located and a needle chamber (11) for mounting the needle structure (4), wherein an inner diameter of the needle cylinder chamber (10) is greater than an inner diameter of the needle chamber (11), and a stepped surface (12) is formed between the needle cylinder chamber (10) and the needle chamber (11).

9. A safety self-destruction syringe according to claim 8, the needle structure (4) includes a needle (45), and when the needle structure (4) is located in the needle cylinder chamber (10), the stepped surface (12) can abut against the needle (45) to limit downward movement of the needle.
